# EUROPÄISCHE PATENTSCHRIFT

(11) **EP 2 456 779 B1**
(45) Veröffentlichungstag und Bekanntmachung des Hinweises auf die Patenterteilung: **15.04.2015**
(21) Anmeldenummer: 10739294.6
(22) Anmeldetag: 07.07.2010
(51) Int. Cl.: C07J 1/00

(54) **17-HYDROXY-17-PENTAFLUORETHYL-ESTRA-4,9(10)-DIEN-11-METHYLENOXYALKYLENARYL-DERIVATE, VERFAHREN ZU IHRER HERSTELLUNG UND IHRE VERWENDUNG ZUR BEHANDLUNG VON KRANKHEITEN**
17-HYDROXY-17-PENTAFLUORETHYL-ESTRA-4,9(10)-DIEN-11-METHYLENE OXYALKYLENE ARYL DERIVATIVES, METHODS FOR THE PRODUCTION THEREOF AND THE USE THEREOF FOR TREATING DISEASES
DÉRIVÉS 17-HYDROXY-17-PENTAFLUOROÉTHYL-ESTRA-4,9(10)-DIÈNE-11-MÉTHYLÉNOXYALCÉNYLARYLE, PROCÉDÉS DE PRÉPARATION ET UTILISATION POUR LE TRAITEMENT DE MALADIES

(30) Priorität: 20.07.2009 DE 102009034366
(43) Veröffentlichungstag der Anmeldung: 30.05.2012
(73) Patentinhaber: Bayer Intellectual Property GmbH, 40789 Monheim (DE)
(72) Erfinder: KLAR, Ulrich, 13503 Berlin (DE); SCHWEDE, Wolfgang, 16548 Glienicke (DE); MÖLLER, Carsten, 10115 Berlin (DE); ROTGERI, Andrea, 13503 Berlin (DE); BONE, Wilhelm, 13467 Berlin (DE)
(74) Vertreter: BIP Patents
(86) Internationale Anmeldenummer: PCT/EP2010/004146
(87) Internationale Veröffentlichungsnummer: WO 2011/009529

(56) Entgegenhaltungen:
- WO-A1-98/34947
- WO-A1-2008/058767

## Beschreibung

Die Erfindung betrifft 17-Hydroxy-17-pentafluorethyl-estra-4,9(10)-dien-11-methylenoxyalkylenaryl-Derivat der Formel I mit Progesteron antagonisierender Wirkung und Verfahren zu ihrer Herstellung, ihre Verwendung zur Behandlung und/oder Prophylaxe von Krankheiten sowie ihre Verwendung zur Herstellung, von Arzneimitteln zur Behandlung und/oder Prophylaxe von Krankheiten, insbesondere von Uterusfibroiden (Myomen, uterine Leiomyome), der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

Diese Verbindungen sind wertvolle pharmazeutische Wirkstoffe. Sie können unter anderem zur Herstellung pharmazeutischer Präparate zur Behandlung von Uterusfibroiden oder der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption verwendet werden. Zur Behandlung von Uterusfibroiden und der Endometriose können die erfindungsgemäßen Verbindungen auch sequentiell in Kombination mit Gestagenen verabreicht werden. In einem solchen Behandlungsregime könnten die erfindungsgemäßen Verbindungen über einen Zeitraum von 1 - 6 Monaten gegeben werden, gefolgt von einer Behandlungspause oder einer sequentiellen Behandlung mit einem Gestagen über einen Zeitraum von 2 - 6 Wochen oder gefolgt von der Behandlung mit einem oralen Kontrazeptivum (OC-Kombinationen) über den gleichen Zeitraum.

Die Wirksamkeit der erfindungsgemäßen Verbindungen als Progesteronrezeptorantagonist wurde in vitro in Transaktivierungstests gezeigt.

Verbindungen mit antagonistischer Wirkung am Progesteronrezeptor (kompetitive Progesteronrezeptorantagonisten) sind erstmals 1982 bekannt (RU 486; EP57115) und seither zahlreich beschrieben worden. Progesteronrezeptorantagonisten mit fluorierter 17α-Seitenkette wurden in WO 98/34947 und Fuhrmann et al., J. Med. Chem. 43, 5010 - 5016 (2000) veröffentlicht.

Die in WO 98/34947 beschriebenen Verbindungen mit fluorierter 17α-Seitenkette weisen im allgemeinen eine sehr starke antagonistische Aktivität am Progesteronrezeptor auf. Sehr potente und daher in WO 98/34947 bevorzugte Verbindungen sind 11β-(4-Acetylphenyl)-20,20,21,21,21-pentafluor-17-hydroxy-19-nor-17α-pregna-4,9-dien-3-on, 11β-(4-Acetylphenyl)-20,20,21,21,21-pentafluor-17-hydroxy-19-nor-17α-pregna-4-en-3-on und 6'-Acetyl-9,11β-dihydro-17β-hydroxy-17α-(1,1,2,2,2-pentafluorethyl)-4'H-naphth [3',2',1':10,9,11]ester-4-en-3-on. Diese Verbindungen werden in vivo in erheblichem Maße in verschiedene Metabolite überführt, die zum Teil-starke, zum Teil geringere pharmakologische Aktivität aufweisen. Der Metabolismus tritt überwiegend am 4-Substituenten des 11-Phenylrestes auf. In WO2008/058767 werden Verbindungen beschrieben, die zumindest zum Teil Metabolite der in WO 98/34947 beschriebenen Verbindungen sind.

Aufgabe der vorliegenden Erfindung ist es, hoch potente kompetitive Progesteronrezeptorantagonisten zur Verfügung zu stellen und damit alternative Behandlungsmöglichkeiten gynäkologischer Erkrankungen zu schaffen.

Es wurde gefunden, dass die erfindungsgemäßen Verbindungen besonders geeignet sind um diese Aufgabe zu lösen.

Die vorliegende Erfindung betrifft 17-Hydroxy-17-pentafluorethyl-estra-4,9(10)-dien-11-methylenoxyalkylenaryl-Derivate mit der allgemeinen chemischen Formel I: 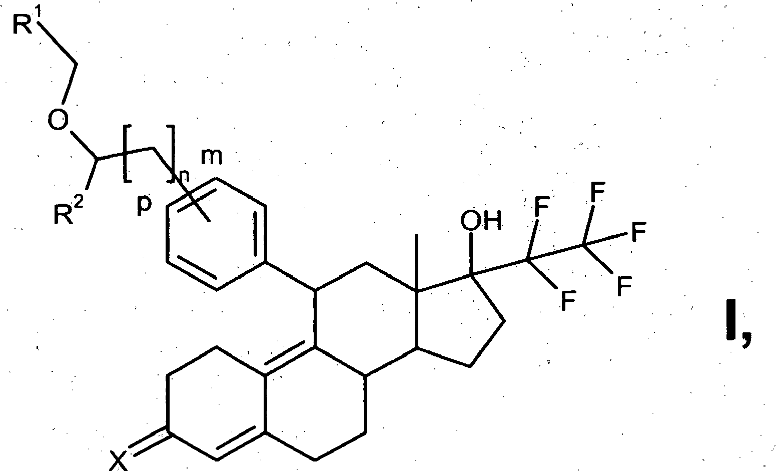 worin
der die Reste R¹ und R² tragende Phenylsubstituent in Position m oder p am Phenylring angeknüpft ist,
n 0 oder 1,
R¹ Wasserstoff, C₁-C₁₀-Alkyl, CH₂OR⁴, CO₂R⁵, CN, Aryl, Heteroaryl,
R² Wasserstoff, C₁-C₁₀-Alkyl,
X Sauerstoff, eine Gruppe NOR³ oder =NNHSO₂R³ bedeuten,
R³ Wasserstoff, C₁-C₁₀-Alkyl, Aryl, C₇-C₂₀-Aralkyl,
R⁴ Wasserstoff, -CH₂-CH₂-OH, CH₂CO₂R⁵,
R⁵ Wasserstoff oder C₁-C₁₀-Alkyl,
bedeuten, sowie deren Stereoisomere, Solvate, Salze oder Solvate der Salze, einschließlich aller Kristallmodifikationen, die α-, β- oder γ-Cyclodextrinclathrate, sowie die mit Liposomen verkapselten Verbindungen bedeuten.

Gemäß einer bevorzugten Ausführungsform der Erfindung ist X ausgewählt aus der Gruppe, umfassend Sauerstoff, NOR³ und NNHSO₂R³. Gemäß einer weiter bevorzugten Ausführungsform der Erfindung steht X für Sauerstoff.

Die erfindungsgemäßen Verbindungen der allgemeinen Formel I können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen einschließlich der Racemate. Aus solchen Mischungen von Enantiomeren und/oder Diastereomeren lassen sich die stereoisomer einheitlichen Bestandteile in bekannter Weise isolieren.

Jeder der genannten Substituenten am Steroid-Grundgerüst kann sowohl in einer α- als auch in einer β-Stellung vorliegen. Außerdem können auch die Substituenten am Steroid-Grundgerüst, die eine Doppelbindung enthalten und in denen die Doppelbindung an jedem Atom mindestens einen Substituenten, der nicht Wasserstoff ist, trägt, sowohl E- als auch Z-konfiguriert vorliegen.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen - wenn eine basische Funktion enthalten ist - Salze mit anorganischen oder organischen Säuren, insbesondere von Mineralsäüren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure oder Benzoesäure.

Physiologisch unbedenkliche Salze der erfindungsgemäßen Verbindungen umfassen - wenn eine saure Funktion enthaltend ist - Alkalimetallsalze, Erdalkalimetallsalze oder Ammoniumsalze, wie sie durch Umsetzung mit entsprechenden anorganischen oder organischen Basen erhalten werden können. Beispielhaft und vorzugsweise seien genannt Alkalimetallsalze (z.B. Natrium- und kaliumsalze), Erdalkalisalze (z.B. Calcium- und Magnesiumsalze) und, Ammoniumsälze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclo-hexylamin, Dimethylaminoethanol, Prokain, Dibenzylamin, N-Methylmorpholin, Arginin, Lysin, Ethylendiamin, N-Methylpiperidin, N-Methyl-glukamin, D-Methyl-glukamin, Ethylglukamin, 1,6-Hexadiamin, Glukosamin, N-Methylglycin, 2-Amino-1,3-propandiol, Trishydroxy-methyl-aminomethan und 1-Amino-2,3,4-butantriol.

Als Solvate werden im Rahmen der Erfindung solche Formen der erfindungsgemäßen Verbindungen bezeichnet, welche in festem oder flüssigem Zustand eine Adduktbildung mit Lösungsmittelmolekülen zeigen. Das Lösungsmittel kann dabei in einem stöchiometrischen oder auch unstöchiometrischen Verhältnis vorliegen. Bei stöchiometrischen Solvaten spricht man auch von Hemi-, (Semi-), Mono-, Sesqui-, Di-, Tri-, Tetra-, Penta-, usw. Solvaten. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfasst Verbindungen, welche während ihrer Verweilzeit im Körper zu erfindungsgemäßen Verbindungen beispielsweise durch enzymatische oder hydrolytische Prozesse umgesetzt werden.

Im Rahmen der vorliegenden Erfindung haben die Substituenten, soweit nicht anders spezifiziert, die folgende Bedeutung:
Alkyl steht für gerad- oder verzweigtkettige Alkylgruppen mit 1-6 Kohlenstoffatomen, wie beispielsweise Methyl, Ethyl, Propyl, Isopropyl, n-Butyl, sec.-Butyl, Isobutyl, tert.-Butyl, n-Pentyl, Isopentyl, Neopentyl Hexyl, Heptyl, Octyl, Nonyl und Decyl.
Aryl steht für einen mono- bis tricyclischen aromatischen, substituierten, oder unsubstituierten carbocyclischen Rest, wie zum Beispiel Phenyl, Naphthyl, die einfach oder mehrfach mit Halogen (F, Cl, Br, I), OH, O-Alkyl, CO₂H, CO₂-Alkyl, NH₂, NH(C₁-C₁₀-Alkyl), N(C₁-C₁₀-Alkyl)₂, insbesondere N(CH₃)₂, NO₂, N₃, CN, C₁-C₁₀-Alkyl, C₁-C₁₀-Perfluor-Alkyl, C₁-C₁₀-Acyl, C₁-C₁₀-Acyloxy-Gruppen, substituiert sein können.
Heteroaryl steht für einen aromatischen, mono- oder bicyclischen Rest mit in der Regel 5 bis 10, vorzugsweise 5 bis 6, Ringatomen und bis zu 5, vorzugsweise bis zu 4 Heteroatomen aus der Reihe S, O und N, beispielhaft und vorzugsweise für Benzofuranyl, Benzothiopheny), Chinolinyl, Furyl, Imidazolyl, Indazolyl, Indolyl, Isochinolinyl Oxazolyl, Pyridazinyl, Pyridyl, Pyrimidyl, Pyrrolyl, Thiazolyl, Thienyl, Pyrazolyl, Isoxazolyl, Pyrazinyl, Chinolyl oder Tetrazolyl, die gegebenenfalls einfach mit C₁-C₄-Alkyl substituiert sein können.
Aralkyl steht für Aralkylgruppen, die im Ring bis zu 14 Kohlenstoffatome, bevorzugt 6-10 Kohlenstoffatome, und in der Alkylkette 1-8, bevorzugt 1-4, Kohlenstoffatome enthalten können. Als Aralkylreste kommen beispielsweise Benzyl, Phenylethyl, Naphthylmethyl, Naphthylethyl, Furylmethyl, Thienylethyl, Pyridylpropyl in Betracht. Die Ringe können einfach oder mehrfach durch Halogen, OH, O-Alkyl, CO₂H, CO₂-Alky NH₂, NH(C₁-C₁₀-Alkyl), N(C₁-C₁₀-Alkyl)₂, NO₂, N₃, CN, C₁-C₂₀-Alkyl, C₁-C₁₀-Perfluor-Alkyl, C₁-C₂₀-Acyl, C₁-C₂₀-Acyloxy-Gruppen substituiert sein.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt sind Verbindungen der Formel (I),
worin
der die Reste R¹ und R² tragende Phenylsubstituent in Position p am Phenylring angeknüpft ist und
- n: 0 oder 1,
- R¹: Wasserstoff, C₁-C₁₀-Alkyl, CH₂OR⁴, CO₂R⁵, CN, Aryl oder Heteroaryl,
- R²: Wasserstoff oder C₁-C₄-Alkyl,
- R³: Wasserstoff, C₁-C₄-Alkyl, Aryl oder C₇-C₁₂-Aralkyl,
- R4: Wasserstoff, -CH₂-CH₂-OH oder CH₂CO₂R⁵,
- R⁵: Wasserstoff oder C₁-C₄-Alkyl und
- X: Sauerstoff, eine Gruppe NOR³ oder =NNHSO₂R³
bedeutet, sowie deren Stereoisomere, Solvate, Salze oder Solvate der Salze, einschließlich aller Kristallmodifikationen, die α-, β- oder γ-Cyclodextrinclathrate, sowie die mit Liposomen verkapselten Verbindungen bedeuten.

Besonders bevorzugt sind Verbindungen der Formel I, worin R¹ Wasserstoff, C₁-C₄-Alkyl, CH₂OR⁴, CO₂R⁵, CN, Aryl-, Heteroaryl bedeutet.

Besonders bevorzugt sind ebenfalls Verbindungen der Formel I worin R²: Wasserstoff, Methyl oder Ethyl bedeutet. Der bevorzugte Rest R² kann dabei sowohl in der R- als auch in der S-Konfiguration, sowie in jedem beliebigen Mischungsverhältnis vorliegen.

Besonders bevorzugt sind weiterhin Verbindungen der Formel I, worin X Sauerstoff bedeutet.

Ganz besonders bevorzugt sind Verbindungen der Formel Ia 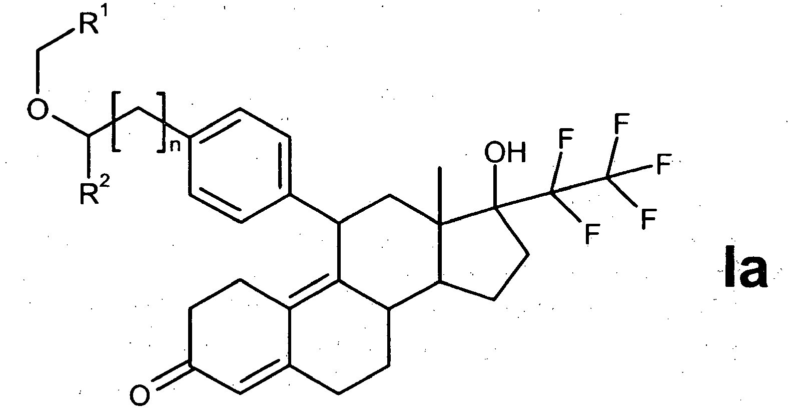 worin
- n: 0 oder 1
- R¹: -H, -CH₂OH, -COOH, .-COOC(CH₃)₃, -CN oder 2-Methyl-benzothiazol-5-yl
- R²: -H oder -CH₃
bedeutet, sowie deren Stereoisomere, Solvate, Salze oder Solvate der Salze einschließlich aller Kristallmodifikationen, die α-, β- oder γ-Cyclodextrinclathrate, sowie die mit Liposomen verkapselten Verbindungen bedeuten..

Die nachstehend genannten Verbindungen sind insbesondere bevorzugt (zusätzlich ist eine Verweisung auf die weiter unten beschriebenen Synthesebeispiele aufgenommen):
(8S,11R,13S,14S,17S)-17-Hydroxy-11-{4-[(1RS)-1-(2-hydroxy-ethoxy)-ethyl]-phenyl}-13-methyl-17-pentafluorethyl-1,2,6,7,8,11,12,13,14,15,16,17-dodecahydro-cyclopenta[a]phenanthren-3-on (Beispiel 1)
(8S,11R,13S,14S,17S)-17-Hydroxy-11-[4-((1RS)-1-methoxy-ethyl)-phenyl]-13-methyl-17-pentafluorethyl-1,2,6,7,8,11,12,13,1,4,15,16,17-dodecahydro-cyclopenta[a]phenanthren-3-on (Beispiel 1)
{(1RS)-1-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-ethoxy}-essigsäure-*tert*-butyl ester (Beispiel 2)
{3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-prop-2-inyloxy}-essigsäure (Beispiel 3)
{2-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethoxy}-essigsäure-tert-butylester (Beispiel 4)
{(RS)-1-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethoxy}-acetonitril (Beispiel 5)
{2-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-ethoxy}-essigsäure (Beispiel 6)
(8S,11R,13S,14S,17S)-17-Hydroxy-11-{4-[2-(2-hydroxy-ethoxy)-ethyl]-phenyl}-13-methyl-17-pentafluorethyl-1,2,6,7,8,11,12,13,14,15,16,17-dodecahydro-cyclopenta[a]phenanthren-3-on (Beispiel 7)
(8S,11R,13S,14S,17S)-17-Hydroxy-13-methyl-11-{4-[(RS)-1-(2-methyl-benzothiazol-5-ylmethoxy)-ethyl]-phenyl}-17-pentafluorethyl-1,2,6,7,8,11,12,13,14,15,16,17-dodecahydro-cyclopenta[a]phenanthren-3-on (Beispiel 8)

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Es wurde gefunden, dass die erfindungsgemäßen Verbindungen bzw. Derivate eine gute, das Progesteron antagonisierende Wirkung aufweisen. In mehreren klinischen Studien wurde gefunden, dass die Behandlung mit Progestronrezeptorantagonisten (Mifepriston, Asoprisnil, Proellex) zu einer signifikanten Schrumpfung von Uterusfibroiden und einer signifikanten Reduktion der mit diesen Uterusfibroiden assoziierten Symptome führen kann. Ferner wurde in klinischen Studien gezeigt, dass unter einer Behandlung mit den genannten Progesteronrezeptorantagonisten auch die von Endometriose verursachten Symptome (insbesondere Schmerzen) deutlich reduziert werden können.

Die Erfindung betrifft ferner Verfahren zur Herstellung von Verbindungen der Formel I, dadurch gekennzeichnet, dass man Verbindungen der Formel II, wie in den Beispielen 1, 2, 4a, 5 und 8 näher beschrieben, verethert, gegebenenfalls in R¹ enthaltene Ester wie in den Beispielen 3 und 6 näher beschrieben, verseift und gegebenfalls in X vorhandene Schutzgruppen, wie in den Beispielen 4 und 7 näher beschrieben, spaltet. Die Herstellung der erfindungsgemäßen Verbindungen kann durch das folgende Syntheseschema verdeutlicht werden.

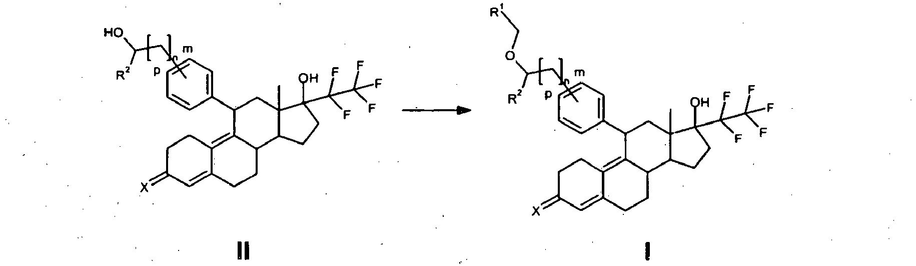

Die erhaltenen Verbindungen der allgemeinen Formel I in denen X für ein Sauerstoffatom steht, können durch Umsetzung mit Hydroxylaminhydrochlorid Alkyloxyamin-hydrochloriden oder Sulfonylhydrazinen in Gegenwart eines tertiären Amins bei Temperaturen zwischen -20 und +40°C in ihre entprechenden E/Z-konfigurierten Oxime oder Sulfonylhydrazone überführt werden (allgemeine Formel I mit X in der Bedeutung von =NOR³, =NNHSO₂R³). Geeignete tertiäre Basen sind beispielsweise Trimethylamin, Triäthylamin, Pyridin, N,N-Dimethylaminopyridin, 1,5-Diazabicyclo[4.3.0]non-5-en (DBN) und 1,5- Diazabicyclo[5.4.0]undec-5-en (DBU), wobei Pyridin bevorzugt ist. Ein analoges Verfahren ist beispielsweise in WO 98/24801 beschrieben.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Soweit die Herstellung der Ausgangsverbindungen hier nicht beschrieben ist, sind diese dem Fachmann bekannt oder analog zu bekannten Verbindungen oder hier beschriebenen Verfahren herstellbar. Die Isomerengemische können nach üblichen Methoden, wie beispielsweise Kristallisation, Chromatographie oder Salzbildung in die einzelnen Verbindungen aufgetrennt werden.

Die Herstellung der Salze erfolgt in üblicher Weise, indem man eine Lösung der Verbindung mit der allgemeinen chemischen Formel I mit der äquivalenten Menge oder einem Überschuss einer Base oder Säure, die sich gegebenenfalls in Lösung befindet, versetzt, gegebenenfalls den Niederschlag abtrennt oder in üblicher Weise die Lösung aufarbeitet.

Die resultierenden Verbindungen der Formel (I) werden gegebenenfalls mit den entsprechenden Lösungsmitteln und/oder Basen oder Säuren zu ihren Solvaten, Salzen und/oder Solvaten der Salze umsetzt.

Die erfindungsgemäßen Verbindungen zeigen ein nicht vorhersehbares, wertvolles pharmakologisches, pharmakokinetisches und pharmakodynamisches Wirkprofil.

Sie eignen sich daher zur Verwendung als Arzneimittel zur Behandlung und/oder Prophylaxe von Krankheiten bei Menschen und Tieren. Die pharmazeutische Wirksamkeit der erfindungsgemäßen Verbindungen lässt sich durch ihre Wirkung als Progesteronrezeptorantagonisten, also ihre antagonisierende Wirkung am Progesteronrezeptor erklären.

Weiterer Gegenstand der vorliegenden Erfindung ist der Einsatz der erfindungsgemäßen Verbindungen zur Behandlung und/oder Prophylaxe von Erkrankungen basierend auf hormonabhängigen hyperproliferativen Prozessen, vorzugsweise von gynäkologischen Krankheiten, insbesondere von Uterusfibroiden, der Endometriose oder von hormonabhängigen Mammakarzinomen.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe von Uterusfibroiden, der Endometriose und von hormonabhängigen Mammakarzinomen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung von 0,1-100 mg der erfindungsgemäßen Verbindungen pro Tag und Patientin bei der Behandlung von Uterusfibroiden oder der Endometriose und für die kontrazeptive Anwendung bzw. von 0,1-500 mg der erfindungsgemäßen Verbindungen pro Tag und Patientin bei Tumor-Erkrankungen (z.B. Menginiome oder hormonabhängige Tumore wie z.B. das Mammakarzinom) und bei der Notfallkontrazeption.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen.

Zur Behandlung von Tumor-Erkrankungen können z.B. die folgenden Wirkstoffe/Wirkstoffklassen entweder gleichzeitig oder sequentiell verabreicht werden: SERMs, SERDs, Antiöstrogene, Aromataseinhibitoren, Kinaseinhibitoren, Angiogeneseinhibitoren und/oder Cytostatika:

Zur Behandlung von Uterusfibroiden oder der Endometriose können die erfindungemäßen Verbindungen gleichzeitig oder sequentiell mit Gestagenen oder Kombinationen aus Östrogenen und Gestagenen kombiniert werden.

In WO 96/15794 (Spicer et al., Balance Pharm. Inc.), WO 96/03130 (Stöckemann et al., Schering AG) und PCT/EP2009/003249 (Möller et al., Bayer Schering Pharma AG) sind Progesteronerezeptorantagonisten/Gestagen-Regime offenbart. Für die Behandlung von Uterusfibroiden und der Endometriose gut geeignet sind - sich gegebenenfalls wiederholende - Regime in denen der Progesteronrezeptorantagonist über einen Zeitraum von zwei bis vier Monaten gegeben wird, gefolgt von der Gabe des Gestagens über einen Zeitraum von ein bis vier Wochen. Besonders gut geeignet ist die - sich gegebenenfalls wiederholende - 84tägige Gabe des Progesteronrezeptorantagonists gefolgt von der 14tägigen Gabe des Gestagens.

Zur Behandlung von mit der Menopause assoziierten Beschwerden kommt eine gleichzeitige oder sequentielle Verabreichung der erfndungsgemäßen Verbindungen z.B. mit SERMs, SERDs und Östrogenen in Betracht.

Selektive Estrogenrezeptormodulatoren (SERM) sind Arzneistoffe, die ihre Wirkung über Estrogenrezeptoren vermitteln aber nicht in allen Geweben eine antagonistische Wirkung hervorrufen. Dazu gehören u.a. Clomifen, Raloxifen, Tamoxifen, Torimifen, Bazedoxifen, Lasofoxifen und Ormeloxifen.

Selektive Estrogenrezeptordestabilisatoren (SERD) sind Arzneistoffe, die den Estrogenrezeptor antagonisieren ('reine Antiöstrogene' ohne östrogene Wirkkomponente) und zu einem partiellen Abbau des Rezeptors führen (beispielsweise Fulvestrant, ZK-703 und ZK-253 [Hoffmann J et al., J Natl Cancer Inst 2004, 96:210-218] sowie in WO 98/007740, WO 99/33855 und WO 03/045972 beschriebene Verbindungen.

Antiöstrogene sind Verbindungen die den Estrogenrezeptor antagonisieren, beispielsweise Fulvestrant.

Aromataseinhibitoren inhibieren das Enzym Aromatase und somit die Aromatisierung von Androgenen in Estrogene. Dazu gehören u.a. Anastrozole, Letrozole, Exemestane, Vorozole, Formestane and Fadrozole.

Kinaseinhibitoren hemmen Enzyme, die einen Phosphatrest von ATP auf andere Substrate, dort insbesondere auf Hydroxygruppen, übertragen, z.B. Sorafenib (Nexavar) oder Imatinib (Gleevec).

Angiogenesehemmer, z.B. Avastin, reduzieren bzw. blockieren die Gefäßneubildung und damit die Durchblutung eines Tumors.

Zytostatika, z.B. cis-Platin, Taxol, Taxotere, Sagopilon, Ixabepilon sind natürliche oder synthetische Substanzen, die Tumorzellen in die Apoptose treiben.

Als Gestagene werden im Sinne vorliegender Erfindung entweder das natürliche Progesteron selbst verstanden oder synthetische Derivate, die wie das Progesteron selbst an den Progesteronrezeptor binden und in Dosierungen, die über der Ovulationshemmdosis liegen, die Ovulation hemmen. Als Beispiele für die synthetischen Derivate seien das Drospirenon, Gestoden, Levonorgestrel, Cyproteronacetat, Desogestrel und 3-Ketodesogestrel, Norethisteron, Norethisteronacetat und das Dienogest genannt.

Bei Kombinationen aus Gestagenen und Östrogenen handelt es sich um die Wirkstoffkombinationen, die in den an sich bekannten oralen Kontrazeptiva, beispielsweise Yasmin, Femovan, Triquilar, Marvelon, YAZ etc., enthalten sind.

Die erfindungsgemäßen Verbindungen können systemisch und/oder lokal wirken. Zu diesem Zweck können sie auf geeignete Weise, wie z.B. oral, intrauterinär, intravaginal, parenteral, pulmonal, nasal, sublingual, lingual, buccal, rectal, dermal, transdermal, conjunctival, otisch oder als Implantat bzw. Stent appliziert werden. Intrauterinär bedeutet dabei insbesondere die Applikation mittels IUS (intrauterine system) oder IUD (intrauterine device). Die intravaginale Applikation kann u.a. mittels IVR (Vaginalring) erfolgen.

Intrauterine oder intravaginale Applikationsformen (vergl. z.B. WO 01/47490, insbesondere Seite 1, Zeile 10 bis Seite 5, Zeile 13 und Seite 7, Zeile 19 bis Seite 58, Zeile 6, oder für Vaginalringe: WO 06/010097, insbesondere Seite 10, Zeile 22 bis Seite 14, Zeile 28) können dabei die erfindungsgemäßen Verbindungen und Nichtsilikon- und/oder Silikonpolymere, insbesondere auch siloxanebasierte Elastomere (vergl. WO 01/47490, insbesondere Seite 7, Zeile 19 - Seite 15, Zeile 15), enthalten. Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln; Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien. Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile, sind Gewichtsteile. Lösungsmittelverhältnisse, Verdünnungsverhältnisse und Konzentrationsangaben von flüssig/flüssig-Lösungen beziehen sich jeweils auf das Volumen.

Die folgenden Beispiele dienen der Erläuterung der Erfindung ohne diese in irgend einer Weise zu beschränken.

### Beispiel 1:

### (8S,11R,13S,14S,17S)-17-Hydroxy-11-{4-[(1RS)-1-(2-hydroxy-ethoxy)-ethyl]-phenyl}-13-methyl-17-pentafluorethyl-1,2,6,7,8,11,12,13,14,15,16,17-dodecahydro-cyclopenta[a]phenanthren-3-on (A) und (8S,11R,13S,14S,17S)-17-Hydroxy-11-[4-((1RS)-1-methoxy-ethyl)-phenyl]-13-methyl-17-pentafluorethyl-1,2,6,7,8,11,12,13,14,15,16,17-dodecahydro-cyclopenta[a]phenanthren-3-on (B)

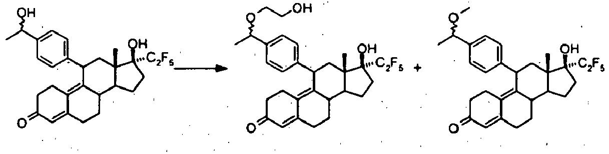

Die Lösung von 1,0 g (1,96 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on (vergl. WO 98/34947, Bsp. 13, Seite 22) in 10 ml Dichlormethan versetzte man mit 0,55 ml Ethylenglykol, 0,43 ml Trimethylorthoformiat, 20 mg p-Toluolsulfonsäure Monohydrat und rührte 48 Stunden bei 23°C. Man versetzte mit gesättigter Natriumhydrogencarbonatlösung, extrahierte mehrfach mit Dichlormethan und trocknete die vereinigten organischen Extrakte über Natriumsulfat. Den nach Filtration und Lösungsmittelabzug erhaltenen Rückstand reinigte man durch Chromatographie. Isoliert wurden 277 mg (26%) der Titelverbindung A sowie 192 mg (19%) der Titelverbindung B, jeweils als farbloser Schaum.

¹H-NMR (CDCl₃) von A: δ = 0,59 (3H), 1,44 (3H), 1,41-1,54 (2H), 1,74-1,85 (3H), 1,97-2,10 (2H), 2,13 (1H), 2,25-2,64 (9H), 2,75 (1H), 3,40 (2H), 3,68 (2H), 4,43 (2H), 5,78 (1H), 7,14 (2H), 7,22 (2H) ppm.

¹H-NMR (CDCl₃) von B: δ = 0,60 (3H), 1,42 (3H), 1,38-1,54 (2H), 1,73-1,87 (3H), 2,01-2,14 (2H), 2,24-2,65 (9H), 2,75 (1H), 3,21 (3H), 4,27 (1H), 4,46 (1H), 5,79 (1H), 7,17 (2H), 7,22 (2H) ppm.

### Beispiel 2

### {(1RS)-1-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethoxy}-essigsäure-tert-butyl ester (A) und {(8S,11R,13S,14S,17S)-11-[4-((RS)-1-tert-Butoxycarbonylmethoxy-ethyl)-phenyl]-13-methyl-3-oxo-17-pentafluorethyl-2,3,6,7,8,11,12,13,14,15,16,17-dodecahydro-1H-cyclopenta[a]phenanthren-17-yloxy}-essigsäure-tert-butyl ester

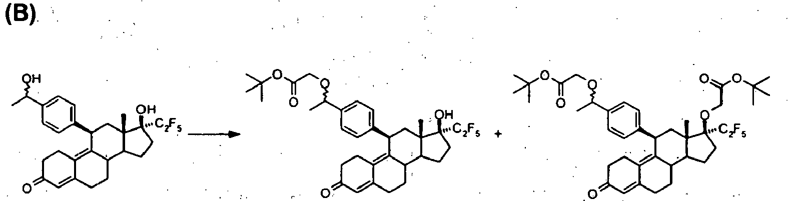

Die Lösung von 500 mg (0,98 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on in 1,5 ml Dichlormethan versetzte man mit 0,81 ml Bromessigsäure-tert.-butylester, 2,3 ml einer 50%igen Kaliumhydroxidlösung, 13 mg Tetrabutylammoniumhydrogensulfat und rührte 2 Stunden bei 23°C. Man verdünnte mit Wasser und Dichlormethan, säuerte durch Zugabe einer 4 molaren Salzsäure an und extrahierte mit Dichlormethan. Die vereinigten organischen Extrakte trocknete man über Natriumsulfat. Den nach Filtration und Lösungsmittelabzug erhaltenen Rückstand reinigte man durch Chromatographie Isoliert wurden 155 mg (21%) der Titelverbindung A sowie 84 mg (14%) der Titelverbindung B, jeweils als farbloser Schaum.

¹H-NMR (CDCl₃) von A: δ = 0,64 (3H), 1,50 (9H), 1,53 (3H), 1,43-1,57 (2H), 1,78-1,91 (3H), 2,06-2,16 (2H), 2,28-2,69 (9H), 2,79 (1H), 3,78 (1 H), 3,92 (1H), 4,49 (1H), 4,56 (1H), 5,83 (1H), 7,19 (2H), 7,29 (2H) ppm.

¹H-NMR (CDCl₃) von B: δ = 0,69 (3H), 1,44 (9H), 1,50 (9H), 1,52 (3H), 1,39-1,59 (2H), 1,77-1,96 (2H), 2,03-2,15 (2H), 2,28-2,85 (10H), 3,76 (1H), 3,91 (1H), 4,11 (2H), 4,47 (1H), 4,55 (1H), 5,82 (1H), 7,20 (2H), 7,28 (2H) ppm.

### Beispiel 3

### {(RS)-1 -[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethoxy}-essigsäure

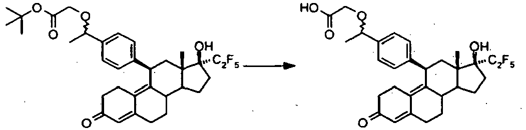

Die Lösung von 57 mg (91 µmol) der nach Beispiel 2 dargestellten Verbindung A in 1 ml Tetrahydrofuran versetzte man mit 0,4 ml einer 5%igen wäßrigen Lithiumhydroxidlösung und rührte 1 Stunde bei 23°C. Man versetzte mit 250 µl einer 1 molaren Natronlauge, 0,5 ml Methanol und rührte weitere 2 Stunden. Man säuerte durch Zugabe einer 1 molaren Salzsäure an, sättigte mit Natriumchlorid, extrahierte mehrfach mit Ethylacetat und trocknete die vereinigten organischen Extrakte über Natriumsulfat. Den nach Filtration und Lösungsmittelabzug erhaltenen Rückstand reinigte man durch Chromatographie. Isoliert wurden 37 mg (71%) der Titelverbindung.
¹H-NMR (CD₃OD): δ = 0,58 (3H), 1,42 (3H), 1,37-1,54 (2H), 1,70-1,82 (3H), 2,09 (1H), 2,18-2,47 (5H), 2,55-2,72 (4H), 2,81 (1H), 3,29 (2H), 3,54-3,74 (2H), 4,45-4,55 (2H), 5,73 (1H), 7,21 (2H), 7,26 (2H) ppm.

### Beispiel 4

### {2-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethoxy}-essigsäure-tert-butylester

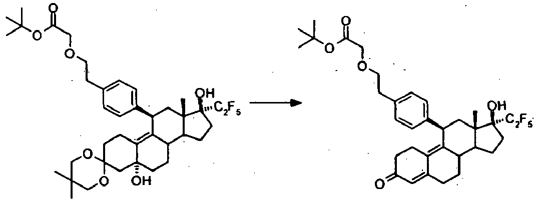

Die Lösung von 90 mg (0,1 mmol) der nach Beispiel 4a dargestellten Verbindung in 2,4 ml Aceton versetzte man mit 160 µl einer 4N Salzsäure und rührte 2 Stunden bei 23°C. Man goss in eine gesättigte Natriumhydrogencarbonatlösung, extrahierte mehrfach mit Dichlormethan, trocknete die vereinigten organischen Extrakte über Natriumsulfat und reinigte den nach Filtration und Lösungsmittelabzug erhaltenen Rückstand durch Chromatographie. Isoliert wurden 37 mg (60%) der Titelverbindungals farbloser Schaum.

¹H-NMR (CDCl₃): δ = 0,59 (3H), 1,47 (9H), 1,40-1,53 (2H), 1,74-1,86 (3H), 2,05, (2H), 2,23-2,63 (9H), 2,72 (1H), 2,91 (2H), 3,72 (2H), 3,96 (2H), 4,42 (1H), 5,77 (1H), 7,09 (2H), 7,15 (2H) ppm.

### Beispiel 4a

{2-[4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1*H*-spiro[cyclopenta[a]phenanthren-3,2'-[1,3]dioxan]-11-yl)-phenyl]-ethoxy}-essigsäure-tert-butylester

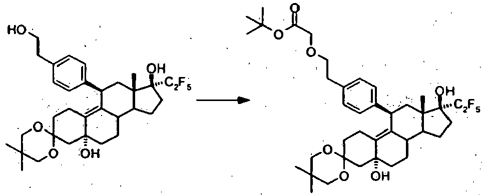

In Analogie zu Beispiel 2 setzte man 250 mg (0,41 mmol) der nach Beispiel 4b dargestellten Verbindung um und isolierte nach Aufarbeitung und Reinigung 181 mg (61%) der Titelverbindung als farblosen Schaum.

### Beispiel 4b

### (5R,8S,11R,13S,14S,17S)-11-[4-(2-Hydroxy-ethyl)-phenyl]-5',5',13-trimethyl-17-pentafluorethyl-1,2,3,4,6,7,8,11,12,13,14,15,16,17-tetradecahydro-1H-spiro[cyclopenta[a]phenanthren-3,2'-[1,3]dioxan]-5,17-diol

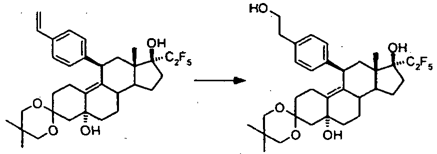

Zu der Lösung von 2,0 g (3,35 mmol) der nach Beispiel 4c dargestellten Verbindung in 20 ml Tetrahydrofuran tropfte man 25 ml einer 0,5 molaren Lösung von 9-Borabicyclo[3.3.1]nonan in Tetrahydrofuran und rührte 4 Stunden bei 23°C. Man kühlte auf 3°C, versetzte mit 11 ml einer 5%igen Natriumhydroxidlösung, 2,9 ml einer 30%igen Wasserstoffperoxidlösung und rührte über Nacht. Man extrahierte mehrfach mit Ethylacetat, wusch mit Wasser und einer gesättigten Natriumthiosulfatlösung und trocknete über Natriumsulfat. Den nach Filtration und Lösungsmittelabzug erhaltenen Rückstand reinigte man durch Chromatographie. Isoliert wurden 1,5 g (73%) der Titelverbindung als farbloser Schaum.

### Beispiel 4c

### (5R,8S,11R,13S,14S,17S)-11-(4-Ethenylphenyl)-5',5',13-trimethyl-17-(pentafluorethyl)-1,2,3,4,6,7,8,11,12,13,14,15,16,17-tetradecahydro-spiro[cyclopenta[a]phenanthren-3,2'-[1,3]dioxan]-5,17-diol

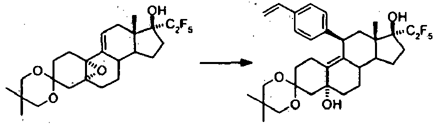

Aus 2,22 g Magnesiumspäne und einer Lösung von 11,95 ml 4-Bromstyrol in 75 ml Tetrahydrofuran stellte man unter leichtem Erwärmen auf 30-50°C und gegebenenfalls unter Zusatz eines Jodkristalls das Grignard-Reagenz her. Man kühlte auf 5°C, versetzte mit 117 mg Kupfer(I)chlorid und tropfte die Lösung von 15 g (30,5 mmol) (5R,8S,10R,13S,14S,17S)-17-(Pentafluorethyl)-5,10-epoxy-5',5',13-trimethyl-1,2,3,4,6,7,8,12,13,14,15,16,17-tridecahydro-spiro[cyclopenta[a]phenanthren-3,2'-[1,3]dioxan]-17-ol, das man nach dem in DE 102006054535 beschriebenen Verfahren herstellte, in 150 ml zu. Man rührte noch 1 Stunde bei 23°C, verdünnte mit Essigsäureethylester und goss in eine gesättigte Ammoniumchloridlösung. Die wäßrige Phase extrahierte man noch mehrfach mit Essigsäureethylester, wusch die vereinigten organischen Extrakte mit gesättigter Natriumchloridlösung und trocknete über Natriumsulfat. Den nach Filtration und Lösungsmittelabzug erhaltenen Feststoff kristallisierte man aus Hexan um und isolierte 16,6 g (91%) der Titelverbindung als farblosen Feststoff.

### Beispiel 5

### {(RS)-1-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethoxy}-acetonitril

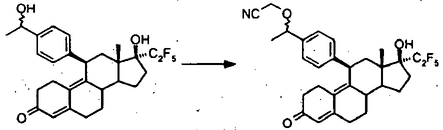

In Analogie zu Beispiel 2 setzte man 250 mg (0,39 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on unter Verwendung von Bromacetonitril um und isolierte nach Aufarbeitung und Reinigung 16 mg (8%) der Titelverbindung als farblosen Schaum.

¹H-NMR (CDCl₃): δ =0,59 (3H), 1,38-1,57 (2H), 1,50 (3H), 1,73-1,87 (3H), 2,02-2,12 (2H), 2,22-2,64 (9H), 2,75 (1H), 3,89+3,94 (1H), 4,14+4,19 (1H), 4,46 (1H), 4,61 (1H), 5,79 (1H), 7,19 (2H), 7,25 (2H) ppm.

### Beispiel 6

### {2-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethoxy}-essigsäure

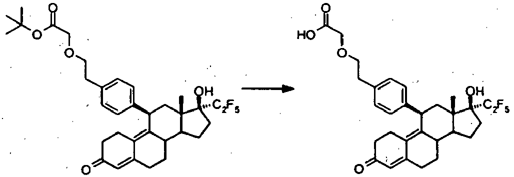

Die Lösung von 32 mg (51 µmol) der nach Beispiel 4 dargestellten Verbindung in 1,4 ml Dichlormethan versetzte man mit 140 µl Trifluoressigsäure und rührte 2,5 Stunden bei 23°C. Man versetzte mit Toluol, engte ein und reinigte den Rückstand durch Chromatographie. Isoliert wurden 25,8 mg (89%) der Titelverbindung als farbloser Schaum.

¹H-NMR (CD₃OD): δ = 0,58 (3H), 1,36-1,54 (2H), 1,70-1,82 (3H), 2,08 (1H), 2,18-2,46 (5H), 2,54-2,90 (7H), 3,64 (2H), 3,84 (2H), 4,48 (1H), 5,73 (1H), 7,13 (2H), 7,17 (2H) ppm.

### Beispiel 7

### (8S,11R,13S,14S,17S)-17-Hydroxy-11-{4-[2-(2-hydroxy-ethoxy)-ethyl]-phenyl}-13-methyl-17-pentafluorethyl-1,2,6,7,8,11,12,13,14,15,16,17-dodecahydrocyclopenta[a] phenanthren-3-on

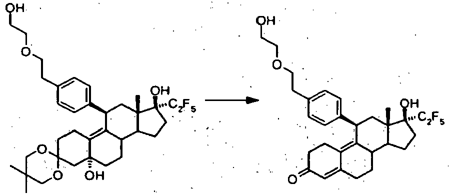

In Analogie zu Beispiel 4 setzte man 64 mg (58 µmol) der nach Beispiel 7a dargestellten Verbindung um und isolierte nach Aufarbeitung und Reinigung 12 mg (38%) der Titelverbindung als farblosen Schaum.

¹H-NMR (CDCl₃): δ = 0,59 (3H), 1,40-1,55 (2H), 1,73-1,92 (4H), 2,05 (1 H), 2,22 (1H), 2,24-2,63 (9H), 2,73 (1H), 2,86 (2H), 3,53 (2H), 3,63-3,74 (4H), 4,43 (1H), 5,78 (1H), 7,09 (2H), 7,13 (2H) ppm.

### Beispiel 7a

### (5R,8S,11R,13S,14S,17S)-11-{4-[2-(2-Hydroxy-ethoxy)-ethyl]-phenyl}-5',5',13-trimethyl-17-pentafluorethyl-1,2,3,4,6,7,8,11,12,13,14,15,16,17-tetradecahydro-1H-spiro[cyclopenta[a] phenanthren-3,2'-[1,3]dioxan]-5,17-diol

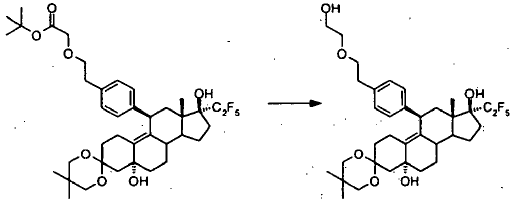

Die Lösung von 83 mg (91 mmol) der nach Beispiel 4a dargestellten Verbindung in 2 ml Toluol versetzte man bei 0°C mit 310 µl, einer 1 molaren Lösung von Diisobutylaluminiumhydrid in Toluol. Nach 1 Stunde goss man in gesättigte Ammoniumchloridlösung, extrahierte mehrfach mit Ethylacetat, wusch die vereinigten organischen Extrakte mit gesättigter Natriumchloridlösung und trocknete über Natriumsulfat. Den nach Filtration und Lösungsinittelabzug erhaltenen Rückstand reinigte man durch Chromatographie. Isoliert wurden 58 mg (97%) der Titelverbindung als farbloser Schaum.

### Beispiel 8

### (8S,11R,13S,14S,17S)-17-Hydroxy-13-methyl-11-{4-[(RS)-1-(2-methyl-benzothiazol-5-ylmethoxy)-ethyl]-phenyl}-17-pentafluorethyl-1,2,6,7,8,11,12,13,14,15,16,17-dodecahydro-cyclopenta[a]phenanthren-3-on

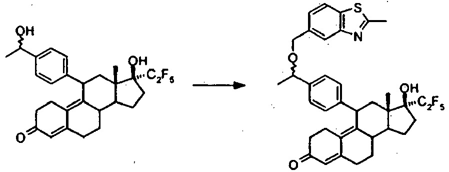

In Analogie zu Beispiel 2 setzte man 500 mg (0,98 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on unter Verwendung von 5-Bromomethyl-2-methyl-benzothiazol um und isolierte nach Aufarbeitung und Reinigung 157 mg (24%) die Titelverbindungen als farblosen Schaum.

### ¹H-NMR (CDCl₃): δ = 0,61+0,63 (3H), 1,47 (3H), 1,39-1,57 (2H), 1,73-1,88 (3H), 2,01-2,14 (2H), 2,26-2,66 (9H), 2,77 (1H), 2,92+2,93 (3H), 4,38-4,55 (4H), 5,79 (1H), 7,17 (2H), 7,25-7,33 (3H), 7,74-7,85 (2H) ppm.

### Beispiel 9: Progesteronerezeptor-antagonistische Wirkung in stabilen Transfektanten humaner Neuroblastoma-Zellen (SK-N-MC Zellen) mit dem humanem Progesteron A- oder Progesteron B-Rezeptor und einem MN-LUC Reporter Konstrukt

SK-N-MC Zellen (humane Neuroblastoma Zellen), die stabil mit Plasmiden transfiziert sind, welche den humanen Progesteronrezeptor-B (pRChPR-B-neo) oder den humanen Progesteronrezeptor-A (pRChPR-A-neo) und ein Reporterkonstrukt (pMMTV-LUC) exprimieren, wurden 24 Stunden entweder in Abwesenheit (Negativkontrolle) oder in Gegenwart von steigenden Mengen der jeweiligen Testverbindung (0,01 nmol/l, 0,1 nmol/l, 1 nmol/l, 10 nmol/l, 100 nmol/l und 1 pmol/l) inkubiert, um die agonistische Wirksamkeit zu bestimmen. Als Positivkontrolle der Reportergeninduktion wurden die Zellen mit dem synthetischen Gestagen Promegeston (0,01 nmol/l, 0,1 nmol/l, 1 nmol/l, 10 nmol/l, 100 nmol/l and 1 pmol/l) behandelt. Zur Bestimmung der antagonistischen Aktivitat wurden die Zellen mit 0,1 nmol/l Promegeston und zusätzlich mit steigenden Mengen der jeweiligen Testverbindung (0,01 nmol/l, 0,1 nmol/l, 1 nmol/l, 10 nmol/l, 100 nmol/l and 1 pmol/l) behandelt. Die Aktivitat des Reportergens LUC (LUC = Luciferase) wurde in den Zellysaten bestimmt und als RLU (relative light units) gemessen. Alle Meßwerte werden angegeben als % Wirksamkeit und als EC₅₀ bzw. IC₅₀ Konzentrationen.
a) agonistische Aktivitat: Keine der genannten Verbindungen zeigt eine agonistische Aktivitat.
b) antagonistische Aktivitat: Alle genannten Verbindungen zeigen eine 100%ige antagonistische Wirksamkeit. Die antagonistische Wirkstärke der Verbindungen ist in Tabelle 1 zusammengefasst.

**Tabelle 1: Antagonistische Wirkstärke der Verbindungen**

| Bsp. | PR-A | PR-B |
|---|---|---|
| | IC₅₀ [nM] | IC₅₀ [nM] |
| 1A | 0,12 | 0,36 |
| 1B | 0,1 | 0,1 |
| 2 | 0,1 | 0,12 |
| 3 | 13 | 9,8 |
| 4 | 0,1 | 0,1 |
| 5 | 0,1 | 0,1 |
| 6 | 3 | 12 |
| 7 | 0,1 | 0,1 |
| 8 | 0,06 | 0,1 |

## Patentansprüche

1. Verbindung der Formel I 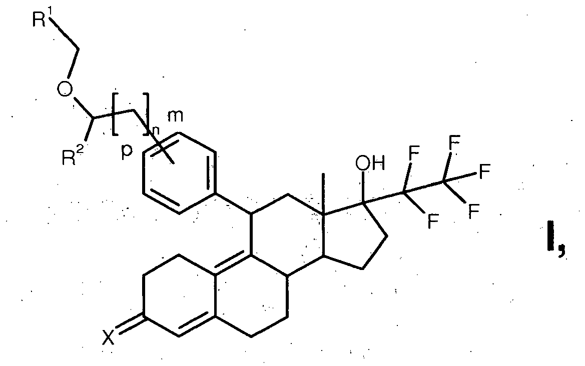 worin
der die Reste R¹ und R² tragende Phenylsubstituent in Position m oder p am Phenylring angeknüpft ist,
n 0 oder 1,
R¹ Wasserstoff, C₁-C₁₀-Alkyl, CH₂OR⁴, CO₂R⁵, CN, Aryl oder Heteroaryl,
R² Wasserstoff oder C₁-C₁₀-Alkyl,
R³ Wasserstoff, C₁-C₁₀-Alkyl, Aryl oder C₇-C₂₀-Aralkyl,
R⁴ Wasserstoff, -CH₂-CH₂-OH oder CH₂CO₂R⁵,
R⁵ Wasserstoff oder C₁-C₁₀-Alkyl;
X Sauerstoff, eine Gruppe NOR³ oder =NNHSO₂R³
bedeutet, sowie deren Stereoisomere, Solvate, Salze oder Solvate der Salze, einschließlich aller Kristallmodifikationen, die α-, β- oder γ-Cyclodextrinclathrate, sowie die mit Liposomen verkapselten Verbindungen bedeuten.

2. Verbindung nach Anspruch 1 worin R¹ Wasserstoff, C₁-C₄-Alkyl, CH₂OR⁴, CO₂R⁵, CN, Aryl oder Heteroaryl bedeutet.

3. Verbindung nach Anspruch 1 worin R² Wasserstoff, Methyl oder Ethyl bedeutet.

4. Verbindung nach Anspruch 1 worin X Sauerstoff bedeutet.

5. Verbindung nach Anspruch 1 worin der die Reste R¹ und R² tragende Phenylsubstituent in Position p am Phenylring angeknüpft ist,
n 0 oder 1,
R¹ Wasserstoff, C₁-C₁₀-Alkyl; CH₂OR¹, CO₂R⁵, CN, Aryl oder Heteroaryl,
R² Wasserstoff oder C₁-C₄-Alkyl,
R³ Wasserstoff, C₁-C₄-Alkyl, Aryl oder C₇-C₁₂-Aralkyl,
R⁴ Wasserstoff; -CH₂-CH₂-OH oder CH₂CO₂R⁵,
R⁵ Wasserstoff oder C₁-C₄-Alkyl,
X Sauerstoff, eine Gruppe NOR³ oder =NNHSO₂R³
bedeutet, sowie deren Stereoisomer, Solvate, Salze oder Solvate der Salze, einschließlich aller Kristallmodifikationen, die α-, β- oder γ-Cyclodextrinclathrate, sowie die mit Liposomen verkapselten Verbindungen bedeuten.

6. Verbindung nach Anspruch 1 der Formel Ia: 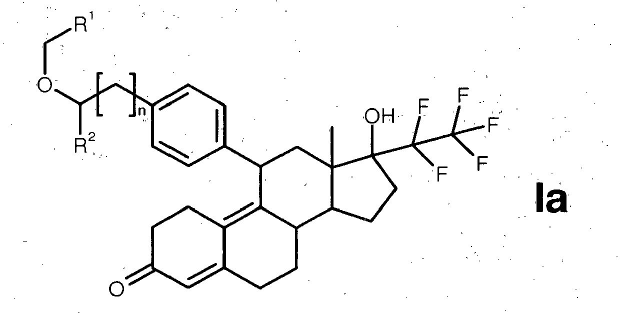 worin
n 0 oder 1
R¹ -H, -CH₂OH, -COOH, -COOC(CH₃)₃, -CN oder 2-Methyl-benzothiazol-5-yl
R² -H oder -CH₃
bedeutet, sowie deren getrennte Stereoisomere, Solvate, Salze oder Solvate der Salze einschließlich aller Kristallmodifikationen.

7. Verbindungen nach Anspruch 1
(8S,11R,13S,14S,17S)-17-Hydroxy-11-{4-[(1RS)-1-(2-hydroxy-ethoxy)-ethyl]-phenyl}-13-methyl-17-pentafluorethyl-1,2,6,7,8,11,12,13,14,15,16,17-dodecahydro-cyclopenta[a]phenanthren-3-on,
(8S,11R,13S,14S,17S)-17-Hydroxy-11-[4-((1RS)-1-methoxy-ethyl)-phenyl]-13-methyl-17-pentafluorethyl-1,2,6,7,8,11,12,13,14,15,16,17-dodecahydrocyclopenta[a]phenanthren-3-on,
{(1RS)-1-[4-((8S,1R,13S,14S,17S)-17-Hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-ethoxy}-essigsäure-tert-butyl ester,
{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-prop-2-inyloxy}-essigsäure,
{2-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethoxy}-essigsäure-tert-butylester,
{(RS)-1-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethoxy}-acetonitril,
{2-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-ethoxy}-essigsäure,
(8S,11R,13S,14S,17S)-17-Hydroxy-11-{4-[2-(2-hydroxy-ethoxy)-ethyl]-phenyl}-13-methyl-17-pentafluorethyl-1,2,6,7,8,11,12,13,14,15,16,17-dodecahydrocyclopenta[a]phenanthren-3-on und/oder
(8S,11R,13S,14S,17S)-17-Hydroxy-13-methyl-11-{4-[(RS)-1-(2-methylbenzothiazol-5-ylmethoxy)-ethyl]-phenyl}-17-pentafluorethyl-1,2,6,7,8,11,12,13,14,15,16,17-dodecahydro-cyclopenta[a]phenanthren-3-on

8. Verbindung, wie in einem der Ansprüche 1 bis 7 definiert, zur Behandlung und/oder Prophylaxe von Krankheiten.

9. Verbindung, wie in einem der Ansprüche 1 bis 7 definiert, zur Behandlung und/oder Prophylaxe von Uterusfibroiden (Myomen, uterine Leiomyome), der Endometriose, schwerer Menstruationsblutungen, Meningiomen, hormonabhängigen Mammakarzinomen und von mit der Menopause assoziierten Beschwerden.

10. Verbindung, wie in einem der Ansprüche 1 bis 7 definiert, zur Fertilitätskontrolle und Notfallkontrazeption.

11. Verwendung einer Verbindung, wie in einem der Ansprüche 1 bis 7 definiert, zur Herstellung eines Arzneimittels zur Behandlung und/oder Prophylaxe von Krankheiten.

12. Verwendung einer Verbindung, wie in einem der Ansprüche 1 bis 7 definiert, zur Herstellung eines Arzneimittels zur Behandlung und/oder Prophylaxe von Uterusfibroiden (Myomen, uterine Leiomyome), der Endometriose, schwerer Menstruationsblutungen, Meningiomen, hormonabhängigen Mammakarzinomen und von mit der Menopause assoziierten Beschwerden.

13. Verwendung einer Verbindung, wie in einem der Ansprüche 1 bis 7 definiert, zur Herstellung eines Mittels zur Fertilitätskontrolle und Notfallkontrazeption.

14. Arzneimittel enthaltend eine Verbindung, wie in einem der Ansprüche 1 bis 7 definiert, in Kombination mit einem oder mehreren inerten, nichttoxischen, pharmazeutisch geeigneten Hilfsstoffen.

15. Arzneimittel nach Anspruch 14, in Kombination mit einem oder mehreren weiteren Wirkstoffen.

16. Arzneimittel nach Anspruch 15, wobei der weitere Wirkstoff ausgewählt ist aus der Gruppe der SERMs, SERDs, Antiöstrogene, Aromataseinhibitoren, Kinaseinhibitoren, Angiogeneseinhibitoren und/oder Cytostatika.

17. Arzneimittel nach Anspruch 15, wobei der weitere Wirkstoff ausgewählt ist aus der Gruppe der Gestagene oder Gestagen/Östrogen-Kombinationen.

18. Arzneimittel nach einem der Ansprüche 15 bis 17, zur Behandlung und Prophylaxe von Uterusfibroiden (Myomen, uterine Leiomyome), der Endometriose, schwerer Menstruationsblutungen, Meningiomen, hormonabhängigen Mammakarzinomen und von mit der Menopause assoziierten Beschwerden.

## Claims

1. Compound of the formula I 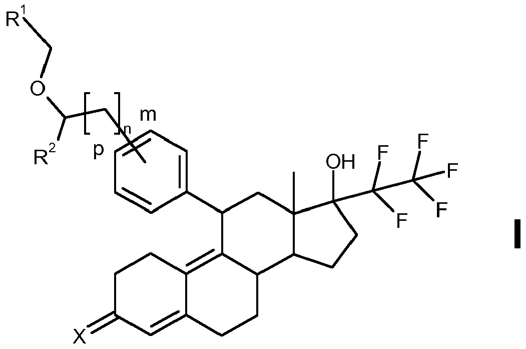 in which
the phenyl substituent bearing the R¹ and R² radicals is joined to the phenyl ring in the m or p position,
n is 0 or 1,
R¹ is hydrogen, C₁-C₁₀-alkyl, CH₂OR⁴, CO₂R⁵, CN, aryl or heteroaryl,
R² is hydrogen or C₁-C₁₀-alkyl,
R³ is hydrogen, C₁-C₁₀-alkyl, aryl or C₇-C₂₀-aralkyl,
R⁴ is hydrogen, -CH₂-CH₂-OH or CH₂CO₂R⁵,
R⁵ is hydrogen or C₁-C₁₀-alkyl,
X is oxygen, an NOR³ or =NNHSO₂R³ group,
and the stereoisomers, solvates, salts or solvates of the salts thereof, including all crystal polymorphs, the α-, β- or γ-cyclodextrin clathrates, and the compounds encapsulated with liposomes.

2. Compound according to Claim 1 in which R¹ is hydrogen, C₁-C₄-alkyl, CH₂OR⁴, CO₂R⁵, CN, aryl or heteroaryl.

3. Compound according to Claim 1 in which R² is hydrogen, methyl or ethyl.

4. Compound according to Claim 1 in which X is oxygen.

5. Compound according to Claim 1 in which the phenyl substituent bearing the R¹ and R² radicals is joined to the phenyl ring in the p position,
n is 0 or 1,
R¹ is hydrogen, C₁-C₁₀-alkyl, CH₂OR⁴, CO₂R⁵, CN, aryl or heteroaryl,
R² is hydrogen or C₁-C₄-alkyl,
R³ is hydrogen, C₁-C₄-alkyl, aryl or C₇-C₁₂-aralkyl,
R⁴ is hydrogen, -CH₂-CH₂-OH or CH₂CO₂R⁵,
R⁵ is hydrogen or C₁-C₄-alkyl,
X is oxygen, an NOR³ or =NNHSO₂R³ group,
and the stereoisomers, solvates, salts or solvates of the salts thereof, including all crystal polymorphs, the α-, β- or γ-cyclodextrin clathrates, and the compounds encapsulated with liposomes.

6. Compound according to Claim 1 of the formula Ia: 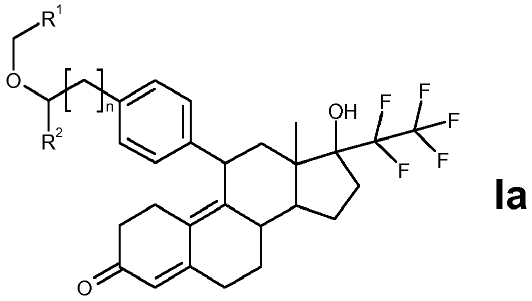 in which
n is 0 or 1
R¹ is -H, -CH₂OH, -COOH, -COOC(CH₃)₃, -CN or 2-methylbenzothiazol-5-yl
R² is -H or -CH₃,
and the separated stereoisomers, solvates, salts or solvates of the salts thereof, including all crystal polymorphs.

7. Compounds according to Claim 1 (8S,11R,13S,14S,17S)-17-hydroxy-11-{4-[(1RS)-1-(2-hydroxyethoxy)ethyl]-phenyl}-13-methyl-17-pentafluoroethyl-1,2,6,7,8,11,12,13,14,15,16,17-dodecahydrocyclopenta[a]phenanthren-3-one, (8S,11R,13S,14S,17S)-17-hydroxy-11-[4-((1RS)-1-methoxyethyl)phenyl]-13-methyl-17-pentafluoroethyl-1,2,6,7,8,11,12,13,14,15,16,17-dodecahydrocyclopenta[a]phenanthren-3-one, {(1RS)-1-[4-((8S,11R,13S,14S,17S)-17-hydroxy-13-methyl-3-oxo-17-pentafluoroethyl-2,3,6,7,8,11,12,13,14,15,16,17-dodecahydro-1H-cyclopenta[a]phenanthren-11-yl)phenyl]ethoxy}acetic acid tert-butyl ester, {3-[4-((8S,11R,13S,14S,17S)-17-hydroxy-13-methyl-3-oxo-17-pentafluoroethyl-2,3,6,7,8,11,12,13,14,15,16,17-dodecahydro-1H-cyclopenta[alphenanthren-11-yl)phenyl]prop-2-ynyloxy}acetic acid, {2-[4-((8S,11R,13S,14S,17S)-17-hydroxy-13-methyl-3-oxo-17-pentafluoroethyl-2,3,6,7,8,11,12,13,14,15,16,17-dodecahydro-1H-cyclopenta[a]phenanthren-11-yl)phenyl]ethoxy}acetic acid tert-butyl ester, {(RS)-1-[4-((8S,11R,13S,14S,17S)-17-hydroxy-13-methyl-3-oxo-17-pentafluoroethyl-2,3,6,7,8,11,12,13,14,15,16,17-dodecahydro-1H-cyclopenta[a]phenanthren-11-yl)phenyl]ethoxy}acetonitrile, {2-[4-((8S,11R,13S,14S,17S)-17-hydroxy-13-methy1-3-oxo-17-pentafluoroethyl-2,3,6,7,8,11,12,13,14,15,16,17-dodecahydro-1*H*-cyclopenta[alphenanthren-11-yl)phenyl]ethoxy}acetic acid, (8S,11R,13S,14S,17S)-17-hydroxy-11-{4-[2-(2-hydroxyethoxy)ethyl]phenyl}-13-methyl-17-pentafluoroethyl-1,2,6,7,8,11,12,13,14,15,16,17-dodecahydro-cyclopenta[a]phenanthren-3-one and/or (8S,11R,13S,14S,17S)-17-hydroxy-13-methyl-11-{4-[(RS)-1-(2-methyl-benzothiazo1-5-ylmethoxy)ethyl]phenyl}-17-pentafluoroethyl-1,2,6,7,8,11,12,13,14,15,16,17-dodecahydrocyclopenta[a]phenanthren-3-one.

8. Compound as defined in any of Claims 1 to 7 for treatment and/or prophylaxis of disorders.

9. Compound as defined in any of Claims 1 to 7 for treatment and/or prophylaxis of fibroids of the uterus (myomas, uterine leiomyomas), endometriosis, heavy menstrual bleeds, meningiomas, hormone-dependent breast cancers and complaints associated with the menopause.

10. Compound as defined in any of Claims 1 to 7 for fertility control and emergency contraception.

11. Use of a compound as defined in any of Claims 1 to 7 for production of a medicament for treatment and/or prophylaxis of disorders.

12. Use of a compound as defined in any of Claims 1 to 7 for production of a medicament for treatment and/or prophylaxis of fibroids of the uterus (myomas, uterine leiomyomas), endometriosis, heavy menstrual bleeds, meningiomas, hormone-dependent breast cancers and complaints associated with the menopause.

13. Use of a compound as defined in any of Claims 1 to 7 for production of a composition for fertility control and emergency contraception.

14. Medicament comprising a compound as defined in any of Claims 1 to 7 in combination with one or more inert nontoxic pharmaceutically suitable excipients.

15. Medicament according to Claim 14, in combination with one or more further active ingredients.

16. Medicament according to Claim 15, wherein the further active ingredient is selected from the group of SERMs, SERDs, antioestrogens, aromatase inhibitors, kinase inhibitors, angiogenesis inhibitors and/or cytostatics.

17. Medicament according to Claim 15, wherein the further active ingredient is selected from the group of the gestagens or gestagen/oestrogen combinations.

18. Medicament according to any of Claims 15 to 17 for treatment and prophylaxis of fibroids of the uterus (myomas, uterine leiomyomas), endometriosis, heavy menstrual bleeds, meningiomas, hormone-dependent breast cancers and complaints associated with the menopause.

## Revendications

1. Composé de formule I 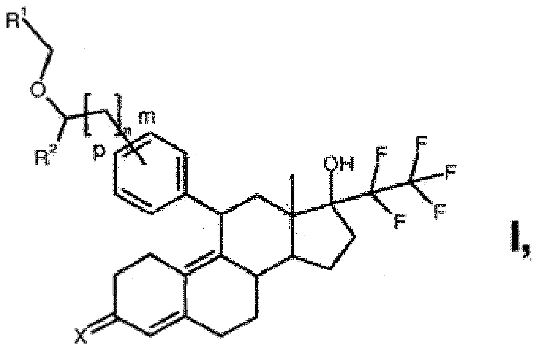 dans laquelle le substituant du phényle portant les radicaux R¹ et R² est lié en position m ou p sur le cycle phényle,
n vaut 0 ou 1 ;
R¹ signifie hydrogène, C₁-C₁₀-alkyle, CH₂OR⁴, CO₂R⁵, CN, aryle ou hétéroaryle,
R² signifie hydrogène ou C₁-C₁₀-alkyle,
R³ signifie hydrogène, C₁-C₁₀-alkyle, aryle ou C₇-C₂₀-aralkyle,
R⁴ signifie hydrogène, -CH₂-CH₂-OH ou CH₂CO₂R⁵,
R⁵ signifie hydrogène ou C₁-C₁₀-alkyle,
X signifie oxygène, un groupe NOR³ ou =NNHSO₂R³
ainsi que ses stéréoisomères, ses solvates, ses sels ou les solvates des sels, y compris toutes les modifications cristallines, les α-cyclodextrine-clathrates, les β-cyclodextrine-clathrates ou les γ-cyclodextrine-clathrates ainsi que les composés encapsulés par des liposomes.

2. Composé selon la revendication 1, dans lequel R¹ signifie hydrogène, C₁-C₄-alkyle, CH₂OR⁴, CO₂R⁵, CN, aryle ou hétéroaryle.

3. Composé selon la revendication 1, dans lequel R² signifie hydrogène, méthyle ou éthyle.

4. Composé selon la revendication 1, dans lequel X signifie oxygène.

5. Composé selon la revendication 1, dans lequel le substituant du phényle portant les radicaux R¹ et R² est lié en position p sur le cycle phényle,
n vaut 0 ou 1,
R¹ signifie hydrogène, C₁-C₁₀-alkyle, CH₂OR⁴, CO₂R⁵, CN, aryle ou hétéroaryle,
R² signifie hydrogène ou C₁-C₄-alkyle ;
R³ signifie hydrogène, C₁-C₄-alkyle, aryle ou C₇-C₁₂-aralkyle,
R⁴ signifie hydrogène, -CH₂-CH₂-OH ou CH₂CO₂R⁵,
R⁵ signifie hydrogène ou C₁-C₄-alkyle ;
X signifie oxygène, un groupe NOR³ ou =NNHSO₂R³
ainsi que ses stéréoisomères, ses solvates, ses sels ou les solvates des sels, y compris toutes les modifications cristallines, les α-cyclodextrine-clathrates, les β-cyclodextrine-clathrates ou les γ-cyclodextrine-clathrates ainsi que les composés encapsulés par des liposomes.

6. Composé selon la revendication 1 de formule Ia : 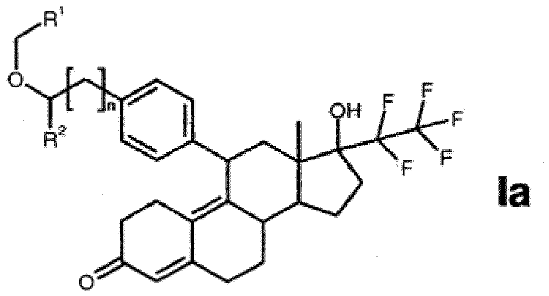 où
n vaut 0 ou 1
R¹ signifie -H, -CH₂OH, -COOH, -COOC(CH₃)₃, -CN ou 2-méthylbenzothiazol-5-yle
R² signifie -H ou -CH₃
ainsi que ses stéréoisomères séparés, ses solvates, ses sels ou les solvates des sels, y compris toutes les modifications cristallines.

7. Composés selon la revendication 1, (8S,11R,13S,14S,17S)-17-hydroxy-11-{4-[(1RS)-1-(2-hydroxyéthoxy)-éthyl]-phényl}-13-méthyl-17-pentafluoroéthyl-1,2,6,7,8,11,12,13,14,15,16,17-dodécahydrocyclopenta[a]phénanthrén-3-one, (8S,11R,13S,14S,17S)-17-hydroxy-11-[4-((1RS)-1-méthoxyéthyl)-phényl]-13-méthyl-17-pentafluoroéthyl-1,2,6,7,8,11,12,13,14,15,16,17-dodécahydrocyclopenta[a]phénanthrén-3-one,
ester tert-butylique de l'acide {(1RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthoxy}-acétique,
acide {3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-prop-2-ynyloxy}-acétique,
ester tert-butylique de l'acide {2-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthoxy}-acétique, {(RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthoxy}-acétonitrile,
acide {2-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthoxy}-acétique,
(8S,11R,13S,14S,17S)-17-hydroxy-11-{4-[2-(2-hydroxyéthoxy)-éthyl]-phényl}-13-méthyl-17-pentafluoroéthyl-1,2,6,7,8,11,12,13,14,15,16,17-dodécahydrocyclopenta[a]phénanthrén-3-one et/ou (8S,11R,13S,14S,17S)-17-hydroxy-13-méthyl-11-{4-[(RS)-1-(2-méthylbenzothiazol-5-ylméthoxy)-éthyl]-phényl}-17-pentafluoroéthyl-1,2,6,7,8,11,12,13,14,15,16,17-dodécahydrocyclopenta[a]phénanthrén-3-one.

8. Composé tel que défini dans l'une quelconque des revendications 1 à 7, destiné au traitement et/ou à la prophylaxie de maladies.

9. Composé tel que défini dans l'une quelconque des revendications 1 à 7, destiné au traitement et/ou à la prophylaxie de fibromes utérins (myomes, léiomyomes utérins), de l'endométriose, de saignements menstruels importants, de méningiomes, de carcinomes mammaires dépendant des hormones et de troubles associés à la ménopause.

10. Composé tel que défini dans l'une quelconque des revendications 1 à 7, destiné au contrôle de la fertilité et à la contraception d'urgence.

11. Utilisation d'un composé tel que défini dans l'une quelconque des revendications 1 à 7, pour la préparation d'un médicament destiné au traitement et/ou à la prophylaxie de maladies.

12. Utilisation d'un composé tel que défini dans l'une quelconque des revendications 1 à 7, pour la préparation d'un médicament destiné au traitement et/ou à la prophylaxie de fibromes utérins (myomes, léiomyomes utérins), de l'endométriose, de saignements menstruels importants, de méningiomes, de carcinomes mammaires dépendant des hormones et de troubles associés à la ménopause.

13. Utilisation d'un composé tel que défini dans l'une quelconque des revendications 1 à 7, pour la préparation d'un agent destiné au contrôle de la fertilité et à la contraception d'urgence.

14. Médicament contenant un composé, tel que défini dans l'une quelconque des revendications 1 à 7, en combinaison avec un ou plusieurs adjuvants inertes, non toxiques, pharmaceutiquement appropriés.

15. Médicament selon la revendication 14, en combinaison avec une ou plusieurs autres substances actives.

16. Médicament selon la revendication 15, l'autre substance active étant choisie dans le groupe des SERM, des SERD, des anti-estrogènes, des inhibiteurs des aromatases, des inhibiteurs des kinases, des inhibiteurs de l'angiogenèse et/ou des cytostatiques.

17. Médicament selon la revendication 15, l'autre substance active étant choisie dans le groupe des gestagènes ou des combinaisons gestagène/estrogène.

18. Médicament selon l'une quelconque des revendications 15 à 17, destiné au traitement et/ou à la prophylaxie de fibromes utérins (myomes, léiomyomes utérins), de l'endométriose, de saignements menstruels importants, de méningiomes, de carcinomes mammaires dépendant des hormones et de troubles associés à la ménopause.
